# EUROPEAN PATENT APPLICATION

(11) **EP 2 884 770 A2**
(43) Date of publication of application: **17.06.2015**
(21) Application number: 14190289.0
(22) Date of filing: 24.10.2014
(51) Int. Cl.: H04R 25/00, A61B 17/58

(54) **Method and device for installing an implant for a bone anchored hearing aid**

(30) Priority: 16.12.2013 EP 13197428
(71) Applicant: Oticon Medical A/S, 2765 Smørum (DK)
(72) Inventor: Jinton, Lars, SE-43144 Mölndal (SE); Johansson, Martin, DK-2765 Smørum (DK)
(74) Representative: Nielsen, Hans Jørgen Vind

(57) **Abstract**

A drill guide for installing an implant in a blind hole in a bone under a soft tissue is disclosed. The drill guide includes a cylindrical portion that includes a cylindrical canal extending along a longitudinal axis of the cylindrical portion. The cylindrical canal is adapted to receive a drill member for drilling the blind hole in the bone. The drill guide further includes a flange at a proximal end section of the drill guide. The drill guide extends perpendicular or substantially perpendicular to the longitudinal axis of the cylindrical portion. The drill guide has a length that is equal or greater than thickness of the soft tissue. A distal end of the drill guide, when inserted in a hole, is adapted to rest against a bone surface that interfaces with the soft tissue.

## Description

### FIELD

The disclosure generally relates to a device and a method for installing an implant. The disclosure more particularly relates to a device and method for installing an implant such as a bone anchored hearing aid, screws and abutments for cranio-facial prostheses and maxillofacial retention systems.

### BACKGROUND

Medical implants such as a bone anchored hearing aids are applied for the rehabilitation of patients suffering from hearing losses for which traditional hearing aids are insufficient. A typical bone anchored hearing aid consists of an external hearing aid provided with a vibrating transducer connected to a skin-penetrating abutment through a coupling. The abutment may have an interconnection to a screw-shaped fixture anchored in the skull bone. The fixture may be made of titanium and may be provided with a flange to prevent the fixture from being pushed through the skull bone when exposed to a sudden accidental impact.

The abutment penetrates the skin and the subcutaneous tissue in order to establish a direct coupling (direct bone conduction) from a hearing aid processor to the skull bone.

The methods for installing bone anchored hearing aid implant systems are moving toward minimally invasive methods that can be performed quickly in order to minimize intra- and post-operative problems, to achieve a predictable outcome, and achieve better cosmetic results.

However, the existing incision techniques are rather complicated and requires that a flap area to be provided by making an incision. Typically, a scalpel is used to make an incision down to the periosteum along a marking of the incision area and to separate the tissue from the underlying periosteum. Further, all subcutaneous tissue in the graft area is separated from the periosteum. Besides the subcutaneous tissue needs to be carefully separated from the skin graft, and all hair follicles needs to be removed. Furthermore, some level of manual skin thinning typically is required to be performed.

Some attempts have been made to avoid the linear incision techniques to install implants for bone anchored hearing aids. Some of these attempts include punch techniques. The techniques apply a standard biopsy punch that is used to provide a circular incision of 5-12 mm.

These techniques are associated with a number of drawbacks. The drill interfaces the soft tissue and hereby introduces the risk of damaging the tissue due to friction, heat and tearing caused by the action of the drill.

These punching techniques apply punching holes larger than 5 mm in order to allow for introducing irrigation fluid (to cool the bone tissue) during the drilling process and also for providing sufficient visibility. These large punch diameters are not optimal for the soft tissue abutment interface. A large circular incision will prolong the healing time and introduce the risk of granulation tissue formation and subsequent infection. Moreover, the skin thickness needs to be determined pre and/or intra operatively.

Thus there is need for a method and device that at least reduces or even eliminates these drawbacks of the prior art.

### SUMMARY

According to an embodiment, the disclosure provides a procedure facilitating a more predictable outcome compared to the incision techniques used today. The procedure includes a less invasive method that requires a shorter healing time when compared to prior art techniques. The method is relatively faster, uncomplicated as well as patient and economy friendly. According to another embodiment, the disclosed method that facilitates irrigation and/ or correct drill depth. Furthermore, the disclosure provides devices that facilitate the method.

### Drill Guide

According to an embodiment, a drill guide for installing an implant in a blind hole in a bone under a soft tissue is disclosed. The drill guide includes a cylindrical portion that includes a cylindrical canal extending along a longitudinal axis of the cylindrical portion. The cylindrical canal is adapted to receive a drill member for drilling the blind hole in the bone. The drill guide further includes a flange at a proximal end section of the drill guide. The flange extends perpendicular or substantially perpendicular to the longitudinal axis of the cylindrical portion. The drill guide has a length that is equal or greater than thickness of the soft tissue. A distal end of the drill guide, when inserted in a hole, is adapted to rest against a bone surface that interfaces with the soft tissue.

The soft tissue includes the skin (including the epidermis and the dermis), the subcutaneous fat and muscle tissue like periost. The blind hole in the underlying bone tissue has a width and depth matching the dimensions of the implant so that the implant may be screwed into the hole.

The substantially perpendicular to the longitudinal axis of the cylindrical portion is defined as minor deviation from alternate perpendicular arrangement. For situations where the flange rests against skin surrounding the incision (cylindrical) hole, such deviation might be useful for allowing the flange to more precisely comply with the skin surface and/ or shape of the patient's head.

The proximal end section is defined as the drill guide section along length of the drill guide that is not surrounded by the soft tissue, when the drill is inserted in the incision hole.

In different embodiments, the flange may include different geometric shapes such as a circular, elliptical, rectangular, etc. Furthermore, the flange may include at least one grip at a periphery of the flange. For example, the flange may include at least two grips preferably at opposite end of each other. The at least one grip is perpendicular or substantially perpendicular to the upper surface of the flange. A grip surface facing outward may include grip enhancers. The grips allow for not only handling the drill guide but also to push the drill guide in an incision hole or for making the incision hole if the drill guide includes blade(s).

It may be useful that the length of the drill guide is at least three times the outer diameter of the drill guide.

In an embodiment, an outer cylindrical wall of the drill guide abuts the soft tissue when inserted and an inner cylindrical wall guides the drill member for generating the blind hole. The use of such drill guide makes it possible to stop the bleeding after punching. The drill guide also protects the soft tissue during a drilling process, where a drill member is inserted into the surgical device. Besides, the drill guide ensures that the correct drill depth is achieved, thus preventing too deep drilling. Furthermore, the drill guide makes it possible to ensure that the drilling is carried out perpendicular to the surface of the bone.

Such arrangement is useful because it fits the structural dimensions of the soft tissue, the implant and drill member of relevance during installation of an implant such as for a bone anchored hearing aid.

### Dual Drill Guide System

In an embodiment, a dual drill guide system is used. The dual drill guide system includes an outer drill guide and an inner drill guide. In case wall member of the outer drill guide bends radially inward, such as because of the pressure applied by soft tissue surrounding the wall member, an inner drill guide may be inserted in the canal of the outer drill guide. This allows the wall members of the outer drill guide to be pushed radially outwards.

The inner diameter of the outer drill guide is slightly larger than the inner or outer diameter of the inner drill guide. The inner drill guide allows for retracting back the outer drill member. Additionally or alternatively, in scenarios where a smaller diameter canal diameter is required after a larger diameter incision hole has already been made, the inner drill member provides such reduced diameter cylindrical canal for guiding the drilling member.

In another embodiment, the dual drill system may be used differently. An inner drill guide is used if an initial drilling process is desired. It may be useful to provide an initial incision hole having a smaller diameter before providing the hole into which the implant has to be installed. An initial hole may be used to test if the bone quality is sufficiently good to achieve a firm attachment of the implant. If the bone quality is sufficiently good, a hole of greater size may be provided by means of the outer drill guide. Accordingly, the inner drill guide may be removed from the outer drill guide.

### Drill Guide including Cooling Channels

In an embodiment, the drill guide includes at least one cooling channel including at least one proximal channel port positioned at the proximal end section and at least one distal channel port positioned at a distal end section of the drill guide. The at least one proximal channel port is adapted to receive a cooling fluid. The at least one cooling channel is adapted to transmit the cooling fluid from the at least one proximal channel port to the at least one distal channel port, which is adapted to expel the cooling fluid out of the at least one cooling channel. The at least one distal channel port is in fluid connection with respective at least one channel. Thus, the cooling channels of the drill guide is adapted to provide adequate irrigation of the bone tissue during a drilling procedure. The skilled person may choose cooling fluid that suits their specific requirement such as an NaCl solution.

The distal end section of the drill guide is defined as the drill guide section along length of the drill guide that is surrounded by the soft tissue, when the drill guide is inserted in the incision hole.

In one embodiment, the drill guide may include one proximal channel port with a peripheral channel at the proximal section of the drill guide, the peripheral channel is in fluid connection with the at least one cooling channel. The proximal channel port receives the cooling fluid, which flows through a peripheral channel and the cooling fluid enters the at least one channel and transmitted to the at least one distal channel port. The peripheral channel is a tubular arrangement that runs along the internal cylindrical wall or external cylindrical wall or sandwiched between the internal and external cylindrical walls of drill guide at the proximal section. In another embodiment, each of the proximal channel port is individually adapted to receive the cooling fluid.

In a first embodiment, the at least one cooling channel is positioned between the inner cylindrical wall and the outer cylindrical wall of the drill guide. This implementation is useful because the size of the drill guide is not altered because the space available between the inner cylindrical wall and the outer cylindrical wall of the drill guide is used. In a second embodiment, the at least one cooling channel is positioned peripheral to the outer cylindrical wall of the drill guide, the outer peripheral surface of the cooling channel abutting the soft tissue. This implementation is useful because a gap between the outer cylindrical wall and the skin tissue because of the peripheral channel reduces transfer of vibrations during drilling to the soft tissue. In a third embodiment, the at least one cooling channel is positioned peripheral to the inner cylindrical wall of the drill guide, the inner peripheral surface of the cooling channel guiding the drill member. This implementation is useful because a gap between the drill member surface and the peripheral channel allows for better air circulation both for heat dissipation and bone debris removal. In yet another embodiment, the cooling channel may be positioned in any combination of the earlier described embodiments. For example, the at least one cooling channels may be positioned peripheral to the inner cylindrical wall and also peripheral to the outer cylindrical wall. In another example, the at least one cooling channel may be positioned peripheral to the inner cylindrical channel, peripheral to the outer cylindrical channel and also between the inner cylindrical wall and the outer cylindrical wall. Other combinations of the earlier described three embodiments are also possible.

According to an embodiment, a drill guide for installing an implant in a blind hole in a bone under a soft tissue is disclosed. The drill guide includes a cylindrical portion that includes a cylindrical canal extending along a longitudinal axis of the cylindrical portion. The cylindrical canal is adapted to receive a drill member for drilling the blind hole in the bone. The drill guide includes the at least one cooling channel having at least one or more combinable features that are described in the earlier sections.

### Drill Guide including Blades

In another embodiment, the distal end section of the drill guide includes a sharp blade at the distal end of the drill guide. Additionally or alternatively, the drill guide may further include spatially separated longitudinal and/ or circular blades along length of the distal end section. The blade and or blades may be selected from a group consisting of regular edge, serrated edge and a combination thereof.

Including blades at the drill guide allow for the drill guide to be used for punching the hole in the soft tissue. Thus, use of separate punch may be avoided and drill guide offers functionalities that include both punching the hole and guiding the drill. Having the drill guide providing dual functionality is particularly useful in arranging the kit because the number of components in the kit is reduced and the kit size is also optimized. In case a separate punch for generating the cylindrical hole in the soft tissue is used, then having the blades at the distal end allows for ensuring that the drill member passes through any residue soft tissue left after the use of the punch with relative ease.

According to an embodiment, a drill guide for installing an implant in a blind hole in a bone under a soft tissue is disclosed. The drill guide includes a cylindrical portion that includes a cylindrical canal extending along a longitudinal axis of the cylindrical portion. The cylindrical canal is adapted to receive a drill member for drilling the blind hole in the bone. The distal end section of the drill guide includes a sharp blade at the distal end with or without spatially separated longitudinal and/ or circular blades along length of the distal end section. The distal end section may also include at least one or more combinable features, as described in any of the preceding paragraphs.

### Drill Guide including Debris Removal

During the drilling of the blind hole, bone dust/ debris is generated. The bone dust or debris rises up along the flutes of the drill member. In order to allow such debris to be removed more efficiently, according to an embodiment, the drill guide includes at least one opening at the proximal end section of the drill guide. During the drilling process, the dust/ bone debris rises along flutes of the drill member and the at least one opening is adapted to allow the risen dust/ bone debris to be expelled from the at least one opening during the drilling process. The at least one opening is positioned along the length of the drill guide such that the at least opening is not surrounded by the soft tissue when the drill guide is positioned in the hole. Such opening may include different shapes such as a slit or hole across the thickness of the walls of the drill guide.

In yet another embodiment, an upper surface of the flange of the drill guide includes a plurality of spatially separated ribs. Each rib of the plurality of ribs includes a first end in immediate proximity to the drill entry port and a second end at a predetermined distance from the drill entry port of the drill guide. The drill entry port is adapted to receive the drill member. Neighboring ribs of the plurality of ribs form a bounded area between the neighboring ribs. The bounded area is adapted to collect the bone dust/ debris that is exported upward from the bone drilling site along the flutes of the drill member and expelled from the drill entry port. The plurality of ribs further allow resting of a drill stop member of the drill member against top of the ribs as opposed to the upper surface of the flange. In conventional drill members without the ribs, the dust/ bone debris collected on the upper surface interferes with the resting of the drill stop member on upper surface of the flange, resulting in inaccurate depth of the blind hole after drilling. Because the dust/ bone debris collected within the bounded region do not interfere with the resting of the drill stop member against the top of the ribs, the blind hole of more accurate and repeatable depth measurement may be generated. (discussed later)

According to an embodiment, a drill guide for installing an implant in a blind hole in a bone under a soft tissue is disclosed. The drill guide includes a cylindrical portion that includes a cylindrical canal extending along a longitudinal axis of the cylindrical portion. The cylindrical canal is adapted to receive a drill member for drilling the blind hole in the bone. The drill guide includes an upper surface of the flange of the drill member includes a plurality of spatially separated ribs and/ or at least one opening at the proximal end section. Furthermore, the drill guide may include one or more combinable features, as described in any of the preceding paragraphs.

### Drill Member

According to another embodiment, a drill member for generating the blind hole is disclosed. The drill member includes at least two flute members extending from close to a drill stop member and twisted along the length of the drill member. The stop member is adapted to abut and rest against the upper surface of the flange or against a plurality of spatially separated ribs of the drill guide. The at least two flute members individually comprise a common distal end and at least one separated distal end. The drill member disclosed here may be used in combination with the disclosed drill guide for implanting the implant.

Additionally or alternatively, it may also be possible to use any other conventionally used drilling device for generating the blind hole. However, it is preferred that the drill member is of a twist drill design with large flutes. Hereby, the method ensures that the bone tissue can be sufficiently cooled down to and also bone fragments may be flushed away from the hole.

In an embodiment, a drill stop member of the drill member is adapted to rest against the upper surface of the flange of the drill guide or top of the plurality of spatially separated ribs during drilling in order to generate desired depth of the blind hole in the bone.

The possibility of having the stop member resting against the upper surface of the flange surface or against the plurality of spatially separated ribs ensures that correct and reproducible blind hole depth is generated. With the distal end of the drill guide inserted in the cylindrical hole and resting against the bone surface that interfaces with the soft tissue along with knowledge of the length of the drill member from the stop member (L_{d}) and length of the drill guide from the upper surface of the flange (L_{f}) or from the top of the pluarlity of spatailly separated ribs (Lg) if the flange includes the plurality of ribs, allows for a simple calculation of determining the depth of the blind hole, i.e. Ld - (Lr or Lg).

Hereby the depth of the blind hole provided in the bone tissue by means of the drill member can be controlled. As described earlier, using the drill member with a stop member is extremely useful and ensures that the drilling process can be controlled and reproduced. Previously known stop members have the shape of flanges extending away from the drill member and adapted to abut the bone tissue directly when a predefined depth has been reached, but by providing a flange which abuts a drill guide, the flange need not enter the hole in the soft tissue, and further, friction heat generated prior to termination of the drilling due to pressure between the flange or stop member will have no effect on tissue, when the stop member abuts the upper flange of the drill guide.

According to another embodiment, the at least two flute members include a common distal end and individual separated distal end. The drill member includes an edge defined by the individual separated distal end of the at least one separated distal end and the common distal end, the edge forms an angle lying between 10-50 degrees with the longitudinal axis of the drill. This arrangement of a common distal end and at least one separated distal end reduces the heat friction generated during initiation of bone drilling process.

According to another embodiment, a middle section width of the drill member is the larger around a middle section of the drill member compared to a distal width proximal to a drill end.

### Healing Cap

According to yet another embodiment, a healing cap adapted to be used post implant is disclosed. The healing cap includes a flexible inner periphery defining an aperture adapted to allow an attachment end of a top portion of an abutment to pass through the aperture. The attachment end is adapted to attach to a processing unit. The healing cap further includes an outer periphery, which in combination with the flexible inner periphery defines an upper surface and a lower surface of the healing cap. The lower surface faces the skin and the upper surface faces away from the skin.

The possibility of attaching the processing unit such as a sound processor when the healing cap is in position allows for fitting the processor to the abutment immediately after the surgery. Thus, time delay with conventional solutions where sound processor is attached only after healing post-surgery has occurred can be avoided.

The dimension of the aperture and flexibility of the inner periphery may be such that the flexible inner periphery temporarily expands when the attachment end is passing through the aperture. Furthermore, when the healing cap is in position, the flexible inner periphery abuts side surface of the abutment by flexibly adjusting the dimension of the flexible inner periphery to comply with the top portion of the abutment.

The healing cap may be made in a semi soft material having a Shore A hardness of 20-95, such as 50-70. The outer periphery of the healing cap may define a shape such as shaped as a circular disk or square disk or rectangular disk, etc. with the aperture being a central circular aperture. The healing cap may be flexible so that the aperture can be enlarged during attachment to the abutment.

According to an embodiment, the healing cap includes a plurality of evenly or unevenly spaced ridges at the lower surface of the healing cap. Each ridge of the plurality of ridges include a distal end that is adapted to abut skin. A plurality of distal ends of the plurality of ridges create pockets of lower surface sections that are at a distance from the skin. These pockets allow for ventilation of the skin, and better retention of a wrap member used for healing process.

In different embodiments, the ridges may be shaped differently such as semi-circular dome with apex distal end of the dome abutting the skin, or a pyramid with a flat apex distal end abutting the skin, or a combination of these or other possible alteration in the shape.

According to another embodiment, the healing cap includes an inner periphery section that is thicker than an outer periphery section of the healing cap. In different embodiments, the variation in thickness from the inner periphery section to the outer periphery section may include a gradual thickness change, a sudden thickness change, a stepped thickness change, and a combination thereof. The difference in the thickness between the inner periphery section and the outer periphery section offers more flexibility at the outer periphery section compared to the inner periphery section. This allows easier maneuverability and handling of the healing cap such as when wrapping around a wrap member between the skin and the healing cap.

The wrap member between the skin and the healing cap offers a slight pressure to the wound. Hereby, optimum conditions for healing may be provided. It may be beneficial that the wrap member has a surface structure allowing air to have access to the area below the wrap member.

This will facilitate the healing process.

It is also conceivable that the lower surface of the healing cap facing the skin may be pre-equipped with the wrap member. The healing cap pre-equipped with the wrap member, when in position i.e. when the flexible inner periphery abuts side surface of the implanted abutment, does not require a separate wrap member to be placed between the lower surface and the skin. In other words, in order to apply the dressing, the healing cap with pre-equipped wrap member is to be simply placed in the position. The pre-equipped healing cap allows for easier, reliable, and faster way of applying the dressing.

The skilled person would appreciate that the disclosed healing cap may also be used for different surgical techniques such as disclosed punch based incision technique, linear incision with skin thinning, linear incision without skin thinning, etc.

### Graded Probe

In an embodiment, a graded probe adapted to measure depth of the hole in the soft tissue is provided. The graded probe includes a plurality of markings representing distance in mm from a distal end of the probe, the distal end being adapted to rest against the bone surface that interfaces with the soft tissue. The markings represents distances such as between 8 mm - 16 mm such as 9 mm, 9.5 mm, 12 mm, 13 mm, 14 mm, 16mm etc.. Other finer markings are also possible and within the scope of this disclosure. When the probe is inserted into the hole and the distal end of the probe rests against bone-skin interface, then the marking aligning with the skin surface gives a reading of the depth of the hole in the soft tissue. The reading thus gives an indication of the thickness of the soft tissue at the punch location, allowing a more accurate choice of abutment of appropriate length. Such probes may be made of stainless steel, plastic or any other suitable material.

### Installation Kit

According to a further embodiment, a kit for installing an implant in a bone under a soft tissue is disclosed. The kit includes at least one of a punch, a drill guide, a drill member, a healing cap, a wrap member, abutment, and an implant. The kit may also include a graded probe. The punch is adapted to punch a cylindrical hole and providing an incision hole in soft tissue by pressuring a sharp blade of a cylindrical hollow punch member through the soft tissue. The drill guide includes a cylindrical canal being adapted to receive a drill member for drilling a blind hole in the bone. Alternatively or additionally, the drill guide includes a cylindrical canal being adapted to receive a drill member for drilling a blind hole in the bone, the drill guide further includes a sharp blade at a distal end with or without spatially separated longitudinal and/ or circular blades along length of the distal end section. The blades are adapted to provide an incision hole in the soft tissue. The drill member is adapted to generate the blind hole while being guided through the cylindrical canal of the drill guide. The healing cap is adapted to allow attaching a processing unit to an abutment with the healing cap abutting the skin. The implant is adapted to be installed in the blind hole in the bone under the soft tissue. The abutment adapted to be installed in the hole abutting the soft tissue. Any or all of the punch, drill guide, drill, and healing cap, included in the kit, may include any combination of features described above. It is comprehensible to have a kit with at least two or at least three or more components. For example, the kit includes the implant and the drill member, or the implant, abutment and drill guide, or the implant, abutment, drill guide, drill member, etc.

It may be beneficial that the kit comprises a drill guide having a length of 15-30 mm such as 20 mm, where the inner diameter of the canal is 2.0-4.9 mm such as 4.9 mm and the outer diameter of the cylindrical portion is 2.5-6.5 mm such as 6.0 mm. The kit may also include a drill member having a diameter of 2.5-6 mm, such as 4.8 mm. Furthermore, the kit may include a punch member having an outer diameter of 3-5 mm such as 4.0 mm or 5 mm. The kit may further include an implant having a threaded portion having an outer diameter of 2.5-5.5 mm such as 4 mm.

The different elements included in the installation kit is adapted to include features of any of the preceding paragraphs.

### Installation Method

According to yet another embodiment, a method for installing an implant is disclosed. The method includes punching a cylindrical hole and providing an incision hole in soft tissue by pressuring a sharp blade of a cylindrical hollow punch member or a sharp blade of the drill guide through a soft tissue. Thereafter, the cylindrical hollow punch member is pulled out of the hole if the cylindrical punch is used in the preceding step. Alternatively, if the drill guide is used to make the hole, the drill guide is retained in position and the drill guide is adapted to guide the drill member. Following this, the punched out soft tissue from the hole is removed. The drill guide, adapted to guide the drill member is inserted into the hole if the cylindrical hollow punch is used to make the incision hole. A blind hole is drill in the underlying bone tissue using the inserted drill member. Lastly, the implant is anchored in the blind hole.

Hereby it is possible to provide a standard procedure with predictable outcome for installing an implant for a bone anchored hearing aid. By applying the method according to the disclosure it is possible to provide a less invasive method that requires shorter healing time compared to the prior art methods. Moreover, the method according to the disclosure is faster, relatively uncomplicated and more patient and economy friendly than the prior art methods.

The method includes the step of providing an incision hole in the soft tissue and a hole in the underlying bone tissue of a subject. The method includes the step of anchoring the implant in the blind hole provided in the bone. This anchoring of the implant may be established by screwing the implant into the bone tissue.

The method comprises the step of punching a cylindrical hole and hereby providing a circular incision in the soft tissue without initially providing a linear incision. Hereby it is possible to reduce the incision area significantly. A prior art incision has a rather large area and the skin graft needs to be sutured. By using the method according to the disclosure, no skin graft needs to be sutured and thus the risk of complications during the healing process is reduced significantly.

When the punching of the cylindrical hole has been carried out, the hole in the bone tissue is provided through a drilling process. Drilling the hole in the underlying bone tissue may be carried out by means of any suitable drilling device. When the cylindrical hole has been provided in the bone structure, the next step of the method is to anchor the implant in the hole.

The anchoring of the implant in the hole in the bone tissue may be carried out by any suitable means. However, in order to provide a reproducible method step it may be an advantage that the implant installation is carried out by means of an electrical drilling equipment. It may be beneficial that low speed setting in the range of 10-20 revolutions per minute (RPM) such as 15 rpm. It may be an advantage that the torque setting is adjusted to suit the quality of the bone tissue. The quality of the bone tissue may be judged by the surgeon during drilling. The punched soft tissue may be picked out with tweezers or another suitable tool. In case the drill guide is used to make the hole in the soft tissue, the drill guide is not withdrawn from the hole and the soft tissue is removed with a pair of tweezers or other suitable tool through the cylindrical canal of the drill guide. It may also be useful for the drill guide to include a scalpel at its distal end. The periost may be removed by means of a small dissector or raspatorium that may be adapted to fit in the drill guide.

It may be a beneficial that the area of the circular incision is equal to or smaller than the cross sectional area of the implant. Hereby it is achieved that the size of the wound can be minimised and thus that optimum healing conditions can be achieved. It may be also be useful that the area of the circular incision is equal to or smaller than the cross sectional area of the abutment of the implant. For example, the punch diameter is 60-100% of the abutment diameter. Hereby it is achieved that the size of the wound can be minimized and thus that optimum healing conditions may be achieved with a proper fit, preferable snug fit, between the skin and the abutment without causing problems like necrosis.

It may be beneficial that the area of the cylindrical hole extends through the soft tissue. Hereby the hole in the underlying bone tissue can be provided by means of drilling directly into the bone tissue without damaging the soft tissue during the drilling procedure.

It may be an advantage that the method comprises the following steps:
a) inserting a drill guide having a centrally arranged canal and a predefined length in the hole;
b) inserting a drill member into the canal of the drill guide;
c) drilling a blind hole in the bone tissue by means of the drill member.

In an embodiment, the step of driving the drill member through is stopped with the stop member of the drill member abuts the upper surface of the drill guide or top of the ribs.

Hereby it is achieved that the drill guide may stop or reduce the bleeding after punching. The drill guide can protect the soft tissue during drilling, ensure that the correct drill depth is achieved (and thus preventing too deep drilling) and facilitate adequate irrigation. Furthermore, the drill guide makes it possible to ensure that the drilling is carried out perpendicular to the surface of the bone and/or skin.

Accordingly, the method according to the disclosure provides a safe and reproducible way of providing a hole in the soft tissue and in the bone tissue and thus to install a bone anchored hearing aid into bone tissue.

It may be useful that the method comprises the steps of applying a drilling device having a drill member and a stop member provided at a predefined distance from the distal end of the drill member.

It may be useful that the method comprises the step of verifying that the geometry and/ or angle and/ or depth of the drilled hole is acceptable. The verification may be carried out by using visual inspection and any suitable measurement tools such as the graded probe.

It may be useful that the blind hole in the underlying bone tissue is made through drilling carried out perpendicular to the surface of the bone. This may be ensured by using a drill guide inserted into the punched hole in the soft tissue and using the drill guide to guide the drill member during the drilling of the bone.

It may be advantageous that the method comprises the step of inserting an expanding drill guide into the drill guide, where the expanding drill guide is configured to radially outwardly push against the inside of the drill guide, as described earlier in the dual-drill system. Hereby the wall member of the drill guide and the soft tissue is pushed in place.

It may be useful that the method comprises the step of cooling down the hole during drilling the hole by supplying a cooling fluid such as sodium chloride dissolved in water to the bone tissue. In one embodiment, this is accomplished by performing a plurality of sequences each comprising a drilling process followed by a drill removal process, where the drill member is removed from the hole in the bone followed by a fluid supplementation process, where a cooling fluid is supplied to the hole in the bone. In another embodiment, where the drill guide with the disclosed cooling channels is used, the irrigation may also be performed during the drilling of the bone. In yet another embodiment, a combination of the two earlier irrigation methods may be used. Hereby the method ensures that the bone tissue can be sufficiently cooled down against overheating and also bone fragments may be flushed away from the hole. When the drill member is removed from the hole, pieces of tissue from the drilling process can be removed e.g. by means of a syringe. It may be an advantage that the method comprises the step of removing pieces of tissue from the drilling process, when the drill member is removed from the hole.

The method may further include the step of attaching a healing cap to the implant. It may be useful that the healing cap is attached between the lower surface of the healing cap and the skin.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. The individual features of each aspect may each be combined with any or all features of the other aspects. These and other aspects, features and/or technical effect will be apparent from and elucidated with reference to the illustrations described hereinafter in which:
Fig. 1A) shows a schematic view of a punch member being used to provide a circular incision according to an embodiment;
Fig. 1B) shows a schematic view of the punch member being removed from the soft tissue after having provided a circular incision according to an embodiment;
Fig. 2A) shows a schematic cross-sectional view of a drill guide arranged in a circular incision hole according to an embodiment;
Fig. 2B) shows a schematic cross-sectional view of a drill member arranged in the drill guide of Fig. 2A) according to an embodiment;
Fig. 3A) shows a schematic cross-sectional view of an outer drill guide arranged in a circular incision hole and an inner drill guide being inserted into the interior of the outer drill guide according to an embodiment;
Fig. 3B) shows a schematic cross-sectional view of the inner drill guide being fully inserted into the interior of the outer drill guide shown in Fig. 3A) according to an embodiment;
Fig. 4A) shows a schematic view of an implant screw arranged above a cylindrical hole provided in the soft tissue and then underlying bone according to an embodiment;
Fig. 4B) shows a schematic cross-sectional view of the implant screw attached to the bone according to an embodiment;
Fig. 5A) shows a schematic cross-section view of at least one cooling channel according to an embodiment;
Fig. 5B) shows a schematic cross-section view of at least one cooling channel according to another embodiment;
Fig. 5C) shows a schematic cross-section view of at least one cooling channel according to yet another embodiment;
Fig. 6A) shows a sharp blade at the distal end of the drill guide according to an embodiment;
Fig. 6B) shows a sharp blade at the distal end of the drill guide according to another embodiment;
Fig. 6C) shows a sharp blade at the distal end of the drill guide according to yet another embodiment;
Fig. 6D) shows a sharp blade at the distal end of the drill guide according to yet another embodiment;
Fig. 6E) shows a sharp blade at the distal end of the drill guide according to yet another embodiment;
Fig. 6F) shows a sharp blade at the distal end of the drill guide according to yet another embodiment;
Fig. 6G) shows circular blades along length of distal end section according to an embodiment;
Fig. 6H) shows longitudinal blades along length of distal end section according to an embodiment;
Fig. 6I) shows circular blades along length of distal end section according to another embodiment;
Fig. 7A) shows at least one opening at the proximal end section of the drill guide according to an embodiment;
Fig. 7B) shows a plurality of spatially separated ribs according to an embodiment;
Fig. 7C) shows a plurality of spatially separated ribs according to another embodiment;
Fig. 8A) shows a drill member according to an embodiment;
Fig. 8B) shows a front view of the drill member according to an embodiment;
Fig. 9A) shows a healing cap according to an embodiment;
Fig. 9B) shows a front view of the healing cap according to an embodiment;
Fig. 9C) shows a lower surface of the healing cap according to an embodiment;
Fig. 10 shows the healing cap in position with an installed abutment according to an embodiment;
Fig. 11 shows a schematic view of the implant screw provided with a healing cap according to an embodiment;
Fig. 12 shows a schematic view of a healing cap being partly exposed from the soft tissue according to an embodiment;
Fig. 13 shows a schematic view of a plurality of sequences carried out when applying the method according to an embodiment; and
Fig. 14 shows a graded probe according to an embodiment.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practised without these specific details.

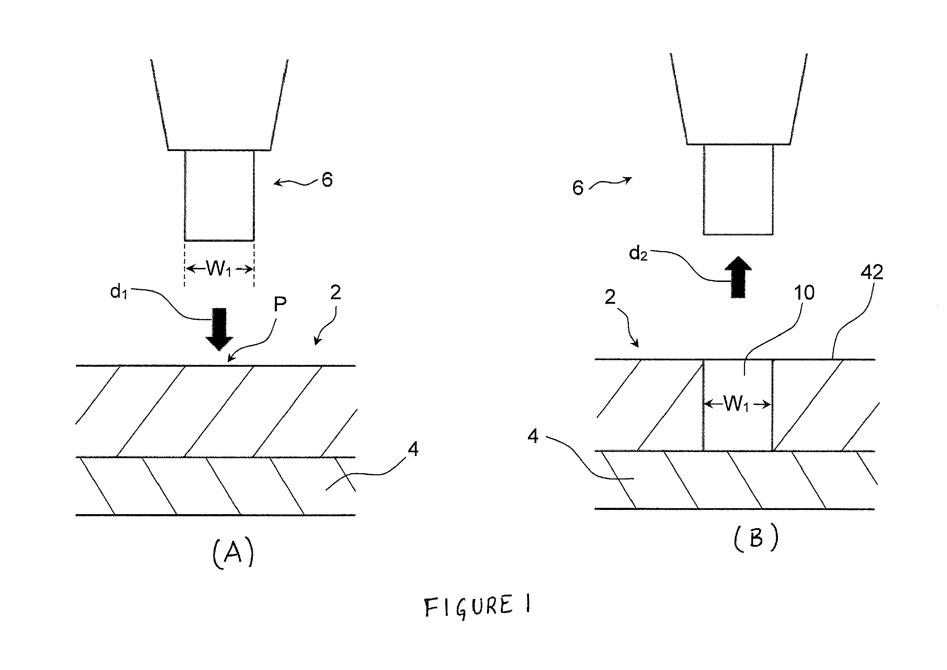

Fig. 1A) shows a schematic view of a punch member 6 being used to provide a circular incision according to an embodiment. The punch member 6 is being moved in the indicated direction d₁ towards the soft tissue 2 of a subject. The punch member 6 may be a standard punch 6 that is supplied sterile. The punch member 6 may be a single use standard punch.

The punch member 6 has a diameter of 5 mm indicated as the width W₁ at Fig. 1A). However, the punch member may have another diameter such as 4.0 mm by way of example. Prior to the punching procedure a suitable position P is chosen and the skin is shaved. Preferably, the skin thickness is measured at the chosen position P e.g. by means of a needle and a ruler, where the needle is inserted into the soft tissue. Hereby it is possible to choose an implant having the most suitable abutment length. The method according to the disclosure may include the step of measuring the bone thickness with ultra sound to ensure that the thickness of the bone tissue 4 is sufficient.

Fig. 1B) shows a schematic view of the punch member 6 being removed, by moving in the indicated direction d2, from the soft tissue 2 after having provided a circular incision hole 10 in the soft tissue 2 according to an embodiment. The soft tissue 2 includes the skin 42 (including the epidermis and the dermis), the subcutaneous fat, and muscle tissue like periost. The punched soft tissue may be picked out with tweezers or another suitable tool (not shown).

After the first punching procedure, another punch may be made in order to ensure that the punch member 6 hits the bone 4 and penetrates the periosteum of the bone 4. Tweezers or another suitable tool may be used to pick out the remaining tissue from the hole 10.

Hereafter the periosteum may be scraped off with a raspatorium or another suitable tool. The shape of the skin 42 surrounding the punched hole 10 may be intact in a time period such as 5 minutes after the punching procedure. Thus, the inner diameter or width W₁ of the punched hole 10 will basically correspond to the width W₁ of the punch member 6. Accordingly, the punched hole 10 may have a diameter and width W₁ of about 4 mm.

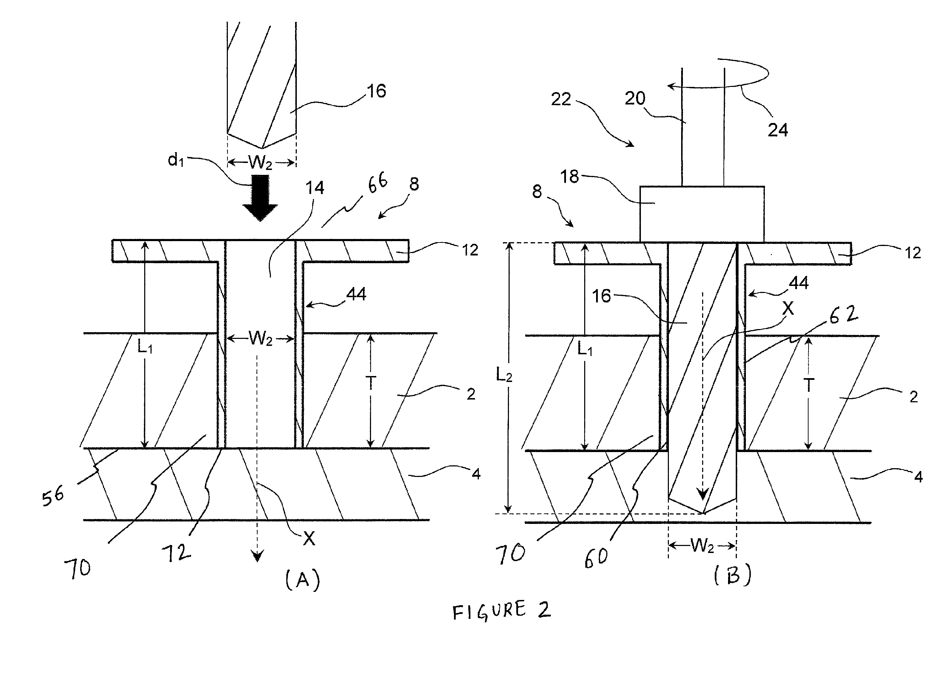

Fig. 2A) shows a schematic view of one of the steps of the method according to the disclosure. Fig. 2A) shows a cross-sectional view of a drill guide 8 arranged in a circular incision hole provided by a technique corresponding to the one shown in Fig. 1B).

The drill guide 8 is used for installing an implant 26 in a blind hole 10' in a bone 4 under a soft tissue 2. The drill guide 8 includes a cylindrical portion 44 provided with a canal 14 extending along the longitudinal axis X of the cylindrical portion 44 of the drill guide 8. The cylindrical canal 14 is adapted to receive a drill member 16 for drilling the blind hole 10' in the bone 4. The drill guide 8 also includes a flange 12, at a proximal end section 66 of the drill guide, extending perpendicular or substantially perpendicular to the longitudinal axis X of the cylindrical portion 44 of the drill guide 8. The length L₁ of the drill guide 8 is greater than the thickness T of the soft tissue 4. The distal end 72 of the drill guide, when inserted in a hole 10, is adapted to rest against a bone surface 56 that interfaces with the soft tissue 2.

When the drill guide 8 is positioned in the circular incision hole, the drill guide 8 rests against a bone surface 56 that interfaces with the soft tissue. In an embodiment, when the drill guide 8 is positioned in the circular incision hole, cooling fluid such as sodium chloride (NaCl) dissolved in water may be introduced into the cylindrical portion 44 of the drill guide 8 e.g. by means of a syringe. Hereby it is achieved that the sodium chloride containing solution is present in the bottom portion of the drill guide 8.

Now the drill guide 8 is ready to receive the drill member 16 that is arranged above the drill guide 8. The drill member 16 needs to be moved in the indicated direction d₁. The drill member 16 may have a diameter or width W₂ of e.g. 3.8 mm.

Fig. 2B) shows a schematic view of the next step of the method according to the disclosure. Fig. 2B) shows a cross-sectional view of a drill device 22 arranged in the drill guide shown in Fig. 2A).

In an embodiment, an outer cylindrical wall 62 of the drill guide 8 abuts the soft tissue 2 when inserted in the incision hole 10 and an inner cylindrical wall 60 guides the drill member 16 for generating the blind hole 10'.

The drill device 22 comprises a drill member 16 extending along the longitudinal axis X of the cylindrical portion 44 of the drill guide 8. The drill device 22 also comprises a stop member 18 and a rod member 20 extending along longitudinal axis X of the cylindrical portion 44 of the drill guide 8. The stop member 18 is arranged between the rod member 20 and the drill member 16. The stop member 16 bears against the flange 12 of the drill guide 8.

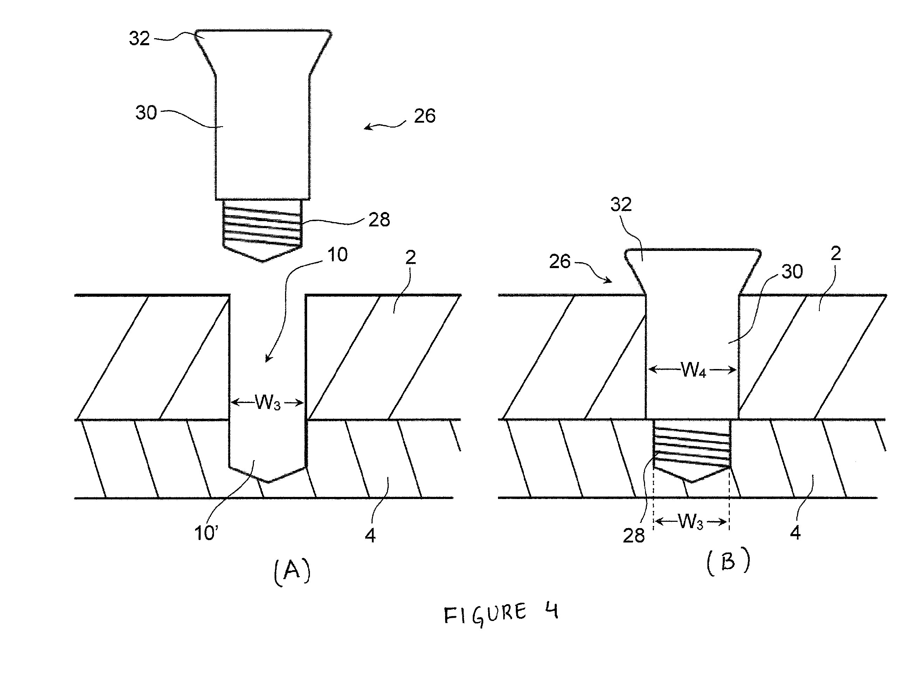

Since the length L₂ of the bore member 16 exceeds the length L₁ of the drill guide 8, the bore member 16 will provide a blind hole 10' in the bone 4. The blind hole 10' is illustrated in Fig. 4A).

The drilling device 22 is rotated in the clockwise direction 24 and thus a blind hole 10' is provided in the bone 4. The diameter or width W₂ of the drill member 16 that corresponds to the inner diameter of the drill guide 8 may be 3.5-4.95 mm such as 4.9 mm by way of example.

The water in the cylindrical portion 44 of the drill guide 8 will ensure that the bone 4 is cooled down during the drilling procedure.

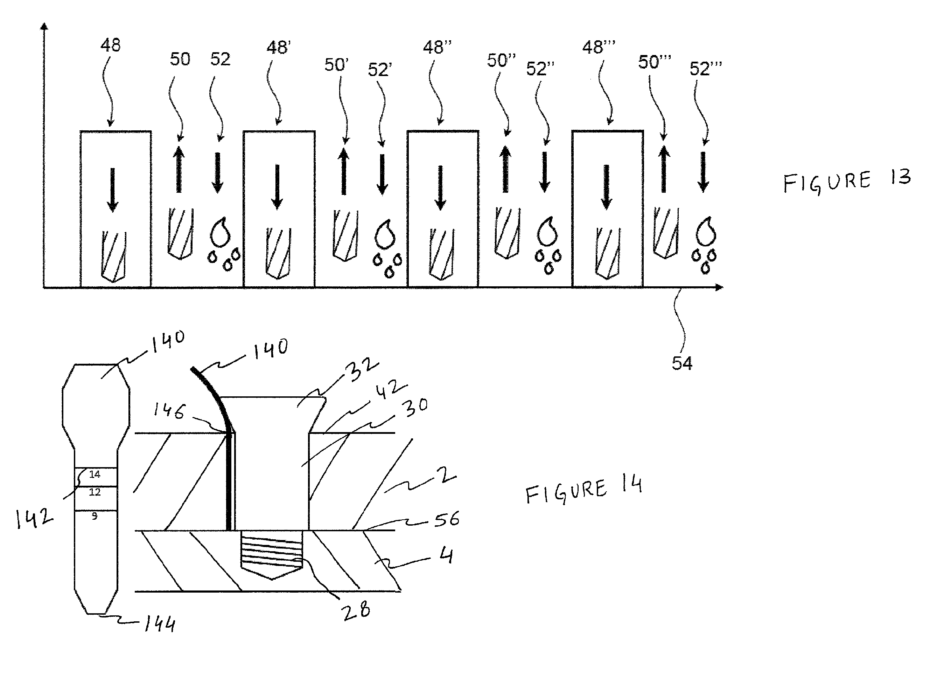

The drill member 16 may be operated at an operational speed of 1000-3000 RPM e.g. 2000 RPM. The drill member 16 may be pulled up once or more to ensure that the bone is cooled down. Hereafter, the drill member 16 is inserted into the bore again and the drilling procedure is continued. This procedure is explained in further detail with reference to Fig. 13.

It may be an advantage that a cooling fluid (e.g. NaCl dissolved in water) is added to the bone tissue 4 via the drill guide 8 every time the drill member 16 is pulled up.

The drill guide 8 stops the bleeding caused by the punching. Moreover, the drill guide 8 protects the soft tissue 2 during the drilling procedure. Further, the length of the drill guide 8 ensures that the drilling depth is correct. The drill guide 8 hereby prevents drilling too deep into the bone tissue 4.

The drill guide 8 may be a single use invasive device e.g. made in a plastic material and produced by an injection moulding process. The drill guide 8 should be sterile when applied.

The drilling device 22 may be a spiral cylindrical drill having a fixed stop member provided at a predefined position at the drill member 16 in order to provide a blind hole having a predefined depth.

When the drill guide 8 is pulled up from the hole 10 pieces of tissue from the drilling process (soft tissue and bone tissue) and the cooling fluid are sucked up by means of a syringe or another suitable means.

In one preferred embodiment of the method according to the disclosure the method comprises the step of alternately drilling a short time period e.g. 1-10 seconds, such as 2-5 seconds and pulling the drill member 16 up in order to cool down the drill member 16. This procedure is repeated several times e.g. 2-10 times such as 3-6 times. This is explained further with reference to Fig. 13.

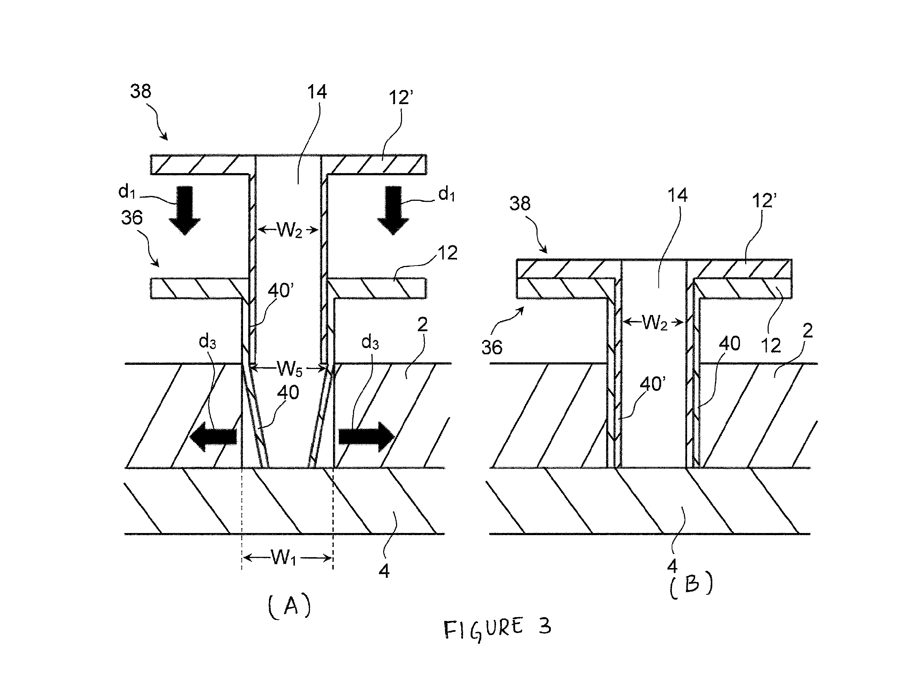

Fig. 3A) shows a schematic cross-sectional view of an outer drill guide 36 arranged in a circular incision hole while an inner drill guide 38 having the function of a spacer being inserted into the interior of the outer drill guide 36.

A lowermost wall member 40 of the outer drill guide 36 is bended radially inward; however, when the inner drill guide 38 is moved downward as indicated with the direction d₁ towards the bone 4, these walls 40 are pushed back radially outwardly as illustrated in Fig. 3B. The arrows indicate the radial directions d₃.

The inner diameter and width W₅ of the outer drill guide 36 is slightly larger than the inner diameter and width W₂ of the inner drill guide 38. The inner drill guide 38 is inserted into the outer drill guide 36 in order to provide a narrow canal 14 to guide a first drill member of a smaller diameter than the drill member 16 shown in Fig. 2B).

The width W₂ of the inner drill guide 38 may also correspond to the width W₂ of the drill member 16 shown in Fig. 2B). The function of the inner drill guide 38 may be to expand the lowermost wall member 40 of the first drill guide 36 if the wall member 40 has been radially inwardly displaced by the surrounding tissue 2. Accordingly, the inner drill guide 38 may be applied to ensure that the canal 14 corresponds to the drill member 16 that is used to provide a blind hole 10' in the bone 4.

Fig. 3B) shows a schematic cross-sectional view of the inner drill guide 38 being fully inserted into the interior of the outer drill guide 36. It can be seen that the lowermost wall member 40 of the first drill guide 36 have been pushed back towards the surrounding soft tissue 2 by the lowermost wall member 40' of the second drill guide 38.

The inner drill guide 38 provides a canal 14 to guide a drill member (not shown). This drill member may have the same or a smaller diameter than the drill member 16 shown in Fig. 2B.

Fig. 4A) shows a schematic view of an implant screw 26 arranged above a cylindrical hole 10 provided in the soft tissue 2 and a further blind hole 10' provided in the underlying bone 4 of a subject. The blind hole 10' extends as an extension of the hole 10. Both holes 10, 10' have been cleaned by sucking up tissue pieces including soft tissue and bone tissue released during the drilling procedure.

The inner width W₃ of the hole 10 basically corresponds to the diameter of the threaded portion 28 of the implant screw 26. The implant screw 26 comprises a top portion 32, an abutment 30 and a threaded portion 28.

Fig. 4B) shows a schematic view of the implant screw 26 inserted into the holes 10 in the soft tissue 2 and in the blind hole 10' in the underlying bone 4.

The implant 26 is inserted into the hole 10 and a torque of sufficient magnitude (e.g. 0.1-10 Nm, such as 0.5 Nm) is applied to fix the implant 26 into the bone 4. The implant 26 may be installed with a relative low rotational speed within the range of 1-100, such as 10-30 RPM by way of example.

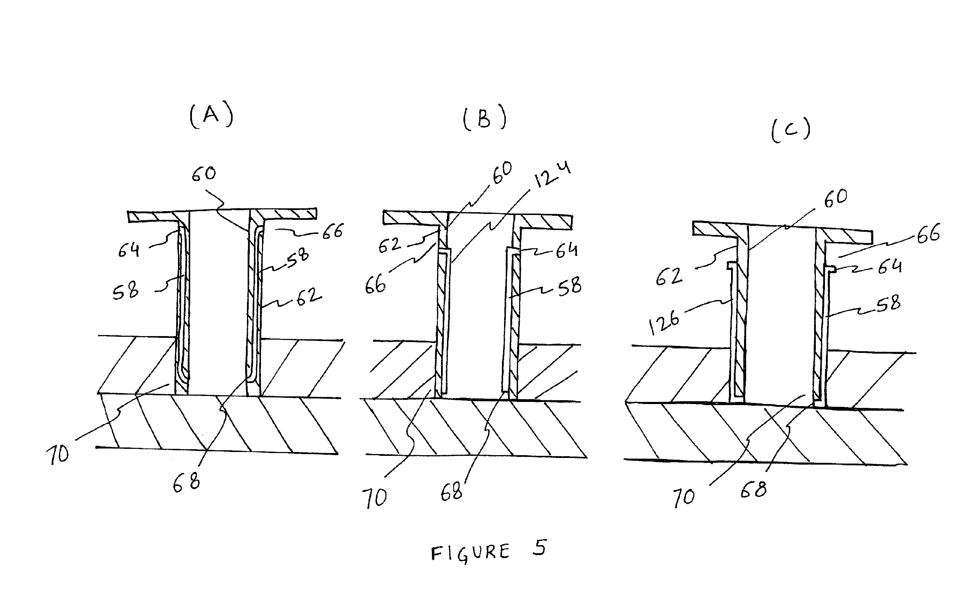

Figs. 5A) - C) show schematic cross-section views of at least one cooling channel according to different embodiments. The at least one cooling channel 58 includes at least one proximal channel port 64 positioned at the proximal end section 66 and at least one distal channel port 68 positioned at a distal end section 70 of the drill guide 8. The at least one proximal channel port 64 is adapted to receive a cooling fluid such as NaCl solution, the at least one cooling channel 58 is adapted to transmit the cooling fluid from the at least one proximal channel port 64 to the at least one distal channel port 68, which is adapted to expel the cooling fluid out of the at least one cooling channel 58.

Fig. 5A) shows a schematic cross-section view of at least one cooling channel according to an embodiment. In this embodiment, the at least one cooling channel 58 is positioned between the inner cylindrical wall 60 and the outer cylindrical wall 62 of the drill guide 8. Fig. 5B) shows a schematic cross-section view of the at least one cooling channel according to another embodiment. The at least one cooling channel is positioned peripheral to the inner cylindrical wall 60 of the drill guide, the inner peripheral surface 124 of the cooling channel guiding the drill member 16. Fig. 5C) shows a schematic cross-section view of at least one cooling channel according to another embodiment. The at least one cooling channel is positioned peripheral to the outer cylindrical wall 62 of the drill guide 8, the outer peripheral surface 126 of the cooling channel abutting the soft tissue.

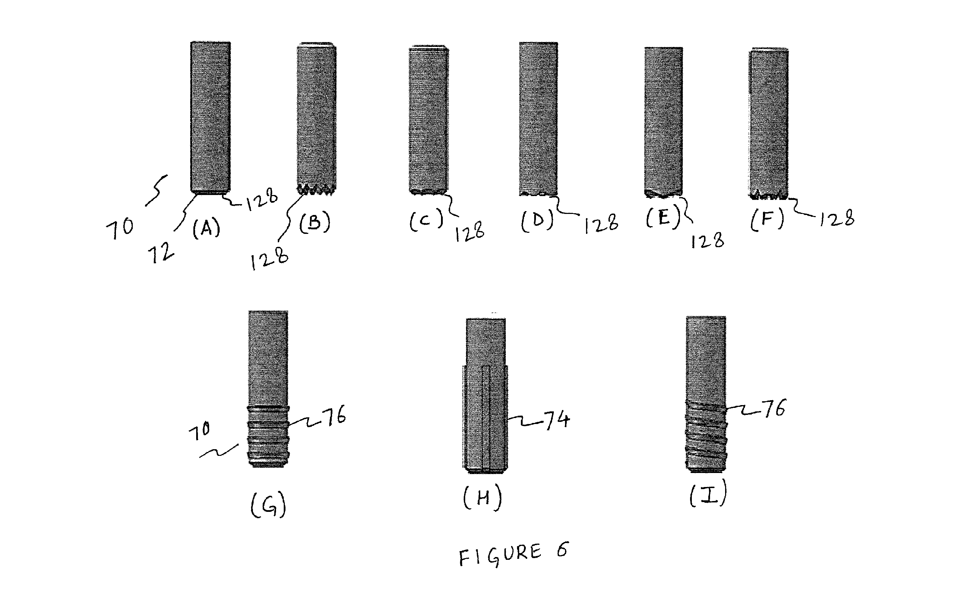

Figs. 6A) - F) show a sharp blade 128 at the distal end 72 of the drill guide 8 according to various embodiments. Fig. 6A) shows the sharp blade 128 at the distal end 72 of the drill guide 8, the sharp blade being a regular edged blade. Fig. 6B)- F) show a sharp edge blade 128 at the distal end 72 of the drill guide 8, the sharp blade being a serrated edge blade.

Fig. 6G) and Fig. I) show circular blades 76 along length of distal end section 70 of the drill guide 8 according to different embodiments. In a first embodiment Fig. 6G), the neighboring circular blades run parallel to each other and to an edge defined by the distal end 72. The circular blades may be equidistant or at different distances along the length of the distal end section 70. In a second embodiment Fig. 6I), the circular blade 76 run parallel to each other but at an angle with the edge defined by the distal end 72. Alternatively, the circular blades may include a single blade that is twisted downwards towards the distal end like a thread of a screw. The circular blades may be equidistant or at different distances along the length of the distal end section 70.

Fig. 6H) shows longitudinal blades 74 along length of distal end section 70 of the drill guide 8 according to an embodiment. In one embodiment, the longitudinal blades 74 are perpendicular or substantially perpendicular to the edge made by the distal end 72 (Fig. 6H). In another embodiment, the longitudinal blades 74 are at an angle to the edge made by the distal end 72 (not shown). In different embodiments, a longitudinal blade may be equidistant from its neighboring longitudinal blades along the periphery of the drill member, or a longitudinal blade may be at different distances from its neighboring longitudinal blades along the periphery of the drill member.

Therefore, in an embodiment, the distal end section 70 of the drill guide 8 comprises a sharp blade 128 at the distal end 72 with or without spatially separated longitudinal 74 and/ or circular 76 blades along length of the distal end section 70, the blade and/ or blades being selected from a group consisting of regular edge, serrated edge and a combination thereof.

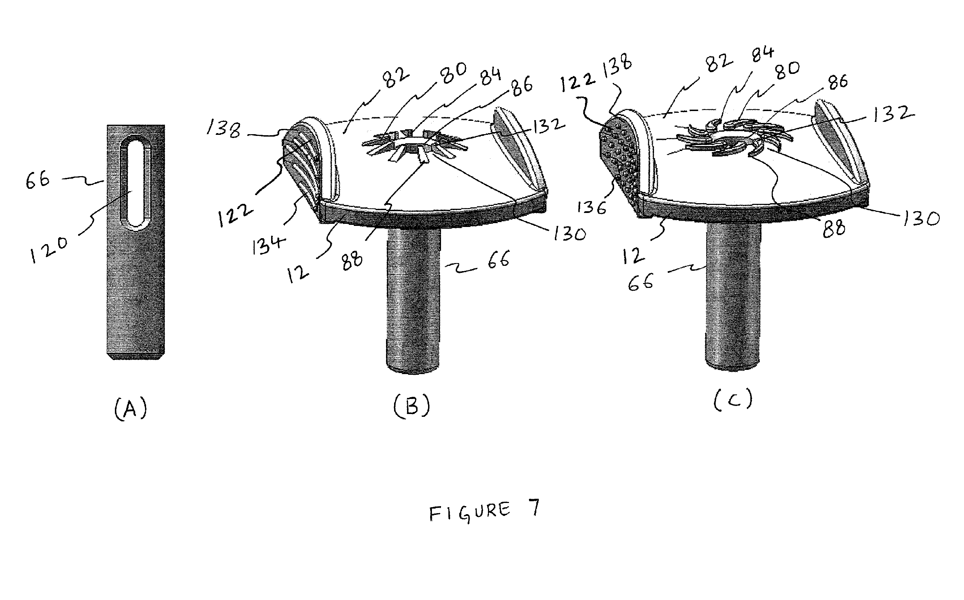

Fig. 7A) shows at least one opening 120 at the proximal end section 66 of the drill guide 8 according to an embodiment. The at least one opening 120 is adapted to expel bone debris during drilling. The at least one opening is positioned along the length of the drill guide such that the at least opening is not surrounded by the soft tissue when the drill guide is positioned in the cylindrical hole. Such opening may include different shapes such as a slit or hole across the thickness of the walls of the drill guide.

Figs. 7B) and 7C) show a plurality of spatially separated ribs according to different embodiments. The plurality of spatially separated ribs 80 on an upper surface 82 of the flange 12 are provided. Each rib of the plurality of ribs 80 comprising a first end 84 in immediate proximity to the drill entry port 86 and a second end 88 at a predetermined distance from the drill entry port 86 of the drill guide 8. The drill entry port 86 is adapted to receive the drill member 16. Neighboring ribs of the plurality of ribs form a bounded area 130 between the neighboring ribs. The bounded area is adapted to collect the bone dust/ debris that is exported upward from the bone drilling site along the flutes of the drill member and expelled from the drill entry port. The plurality of spatially separated ribs 80 further allow resting of a drill stop member (18, 92) of the drill member 16 against a top 132 of the ribs as opposed to the upper surface 82 of the flange.

In an embodiment, the flange may include at least one grip 122 at a periphery of the flange. For example, the flange may include at least two grips preferably at opposite end of each other, as illustrated in Figs. 7B) and 7C). The grips allow for not only handling the drill guide 8 but also to push the drill guide 8 in an incision hole 10 or for making the incision hole 10 if the drill member includes blade(s), such as the ones illustrated in Figs. 6A) - 6I). Additionally, the grip surface 138 may include grip enhancers (134, 136) such as protrusions including longitudinal protrusions (134, Fig. 7B) or circular protrusions (136, Fig. 7C) extending from the grip surface 138, allowing better grip of the at least one grip 122.

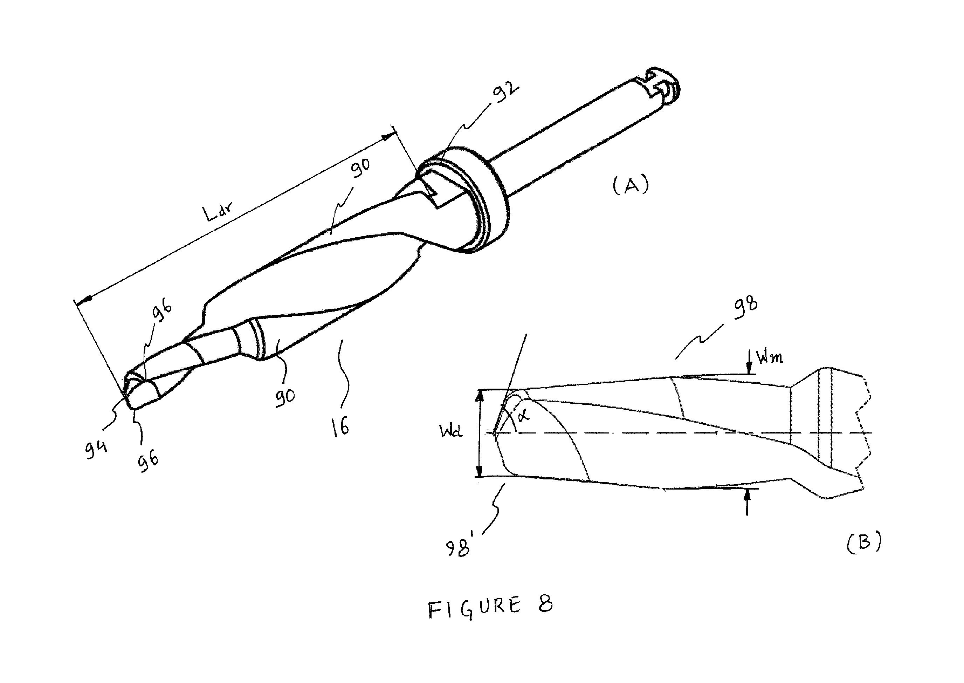

Fig. 8A) shows a drill member 16 according to an embodiment and Fig. 8B) shows a front view of the drill member according to an embodiment. The drill member 16 includes at least two flute members 90 extending from close to a drill stop member 92 (in earlier section, the stop member is shown as numeral 18). The at least two flute members are twisted along a length L_{dr} of the drill member 16 and the stop member 92 is adapted to abut and rest against the upper surface 82 of the flange 12 or against the plurality of spatially separated ribs 80 of the drill guide 8. The at least two flute members 90 individually comprise a common distal end 94 and at least one separated distal end 96.

In an embodiment, the drill member includes an edge defined by the individual separated distal end 96 of the at least one separated distal end and the common distal end. The edge forms an angle α with the longitudinal axis of the drill, the angle lies between 10-50 degrees.

In another embodiment, a middle section width Wm of the drill member 16 is the larger around a middle section 98 of the drill member 16 compared to a distal width Wd that is proximal to a drill end 98'.

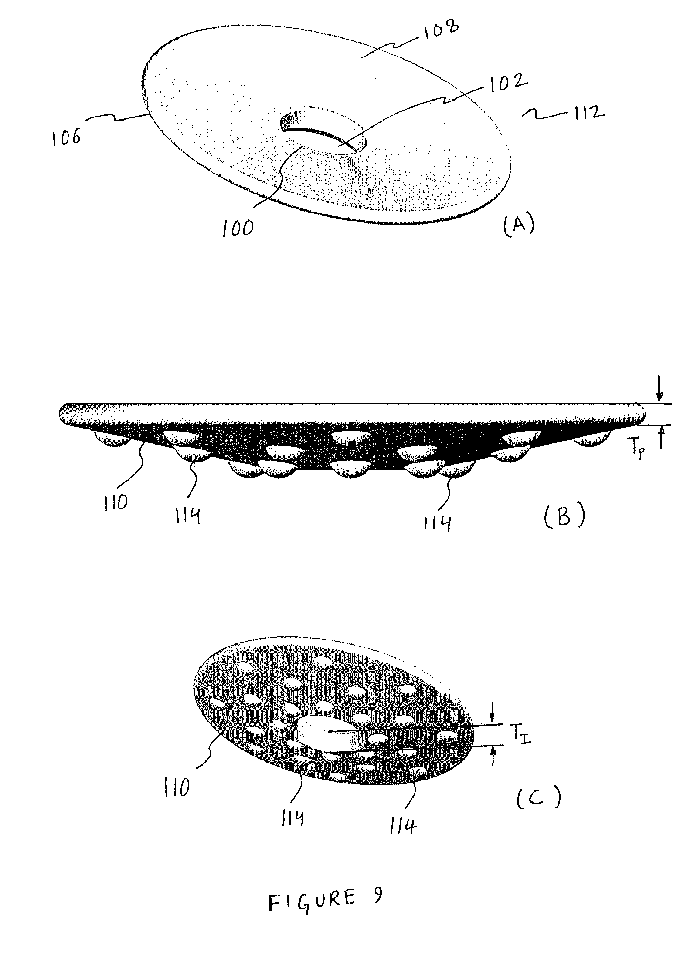

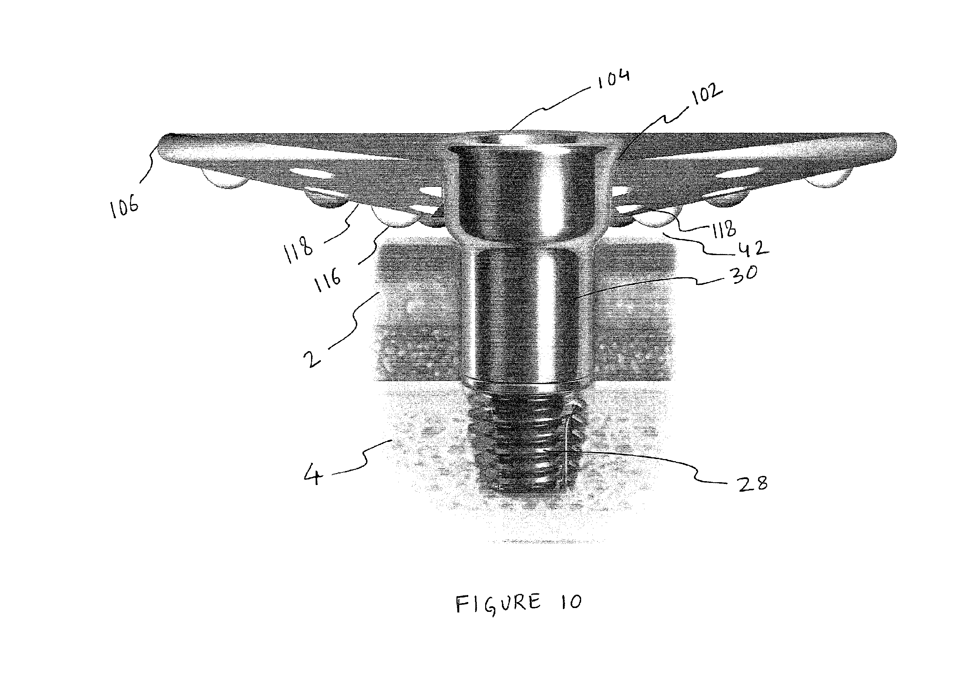

Fig. 9A) shows a healing cap 112 according to an embodiment, Fig. 9B) shows a front view of the healing cap 112 according to an embodiment; and Fig. 9C) shows a lower surface 110 of the healing cap 112 according to an embodiment. Fig. 10 shows the healing cap 112 in position with an installed abutment 30 according to an embodiment.

The healing cap 112 includes a flexible inner periphery 100 defining an aperture 102 adapted to allow an attachment end 104 of a top portion 32 of an abutment 30 to pass through the aperture 102. The attachment end 104 is adapted to attach to a processing unit such as a sound processor of a bone anchored hearing aid. The healing cap further includes an outer periphery 106, which in combination with the flexible inner periphery 100 defines an upper surface 108 and a lower surface 110 of the healing cap 112. The lower surface 110 faces the skin 42 and the upper surface facing away from the skin 42 (see Fig. 10).

In an embodiment, the healing cap 112 includes a plurality of evenly or unevenly spaced ridges 114 at the lower surface 110 of the healing cap 112 (see Fig. 9). A distal end 116 of at least the plurality of ridges 114 that are proximal to the aperture 102 are adapted to abut the skin 42 and to create pockets 118 of lower surface sections that are at a distance from the skin (see Fig. 10).

In another embodiment, as shown in Figs. 9B) and 9C), the healing cap 112 includes an inner periphery section 122 that is thicker than an outer periphery section 124, i.e. T_{P} > T_{I}. The variation in thickness is from a group consisting of a gradual thickness change, a sudden thickness change, a stepped thickness change, and a combination thereof.

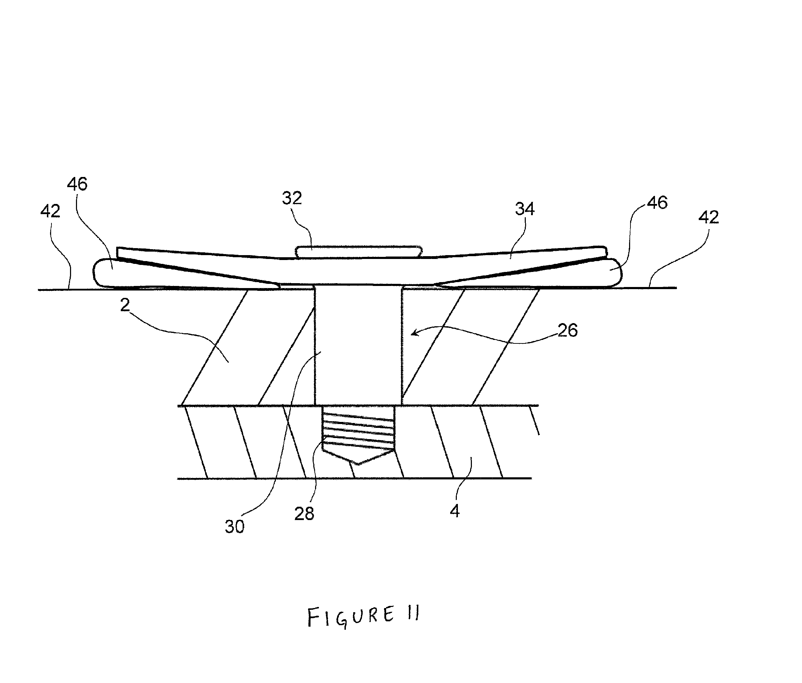

Fig. 11 shows a schematic view of the implant screw 26 provided with a healing cap 34. The method according to the disclosure may comprise the step of providing a healing cap 34 to the implant screw 26 after the implant screw has been inserted into the bone 4.

The healing cap 34 is attached to the uppermost portion of the abutment 30 just below the top portion 32. The healing cap 34 may be made in a semi soft material having a Shore A hardness of 20-75, such as 40-60. The healing cap 34 may be shaped as a circular disk with a central circular aperture. The healing cap 34 may be flexible so that the aperture can be enlarged during attachment to the abutment 30.

A wrap member 46 may be provided between the skin 42 and the healing cap 34 in order to provide a slight pressure to the wound for a predefined time period e.g. 1-2 weeks, such as ten days. The wrap member 46 may have a surface structure allowing air to have access to the area below the wrap member 46.

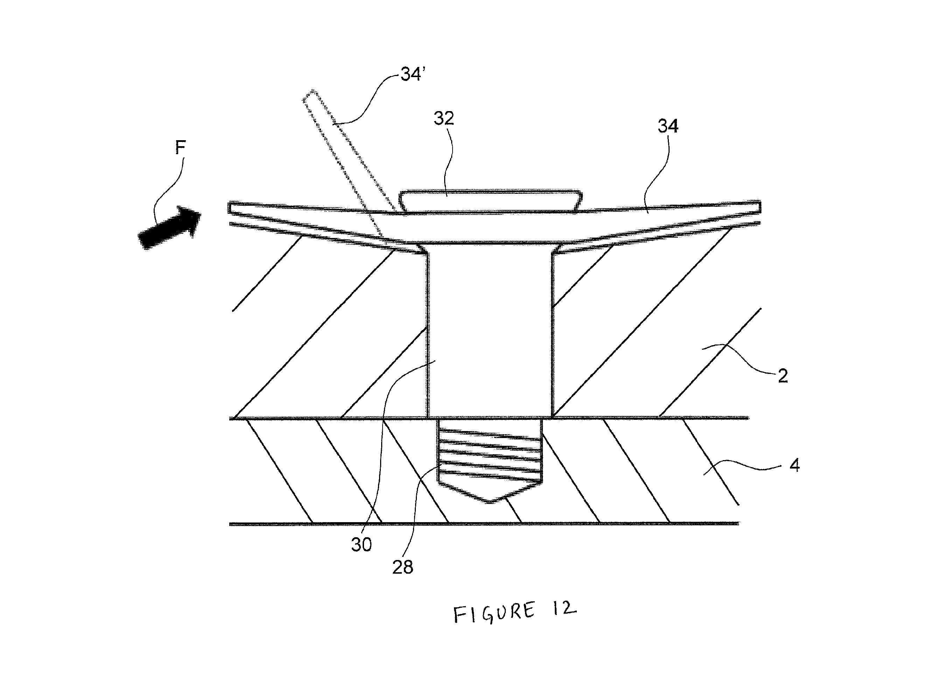

Fig. 12 shows a schematic view of a healing cap 34 being partly exposed from the soft tissue 2 surrounding the abutment 30. Fig. 6 shows that a portion 34' of the healing cap 34 is moved upwards when a force F is applied. This healing cap 34 may be moved upwards in order to provide a wrap member 46 between the skin 42 and the healing cap 34. This may be done in order to provide a slight pressure to the wound (created by the punching process) for a predefined time period (e.g. 1-2 weeks, such as ten days). The wrap member 46 may have a surface structure allowing air to have access to the area below the wrap member 46.

The skilled person would appreciate that in one implementation, the features of the healing cap 112 and the healing cap 34 are combinable.

Fig. 13 shows a schematic view of a plurality of sequence of the method according to the disclosure. The sequences are plotted against time 54. Each sequence comprises a drilling process 48, 48', 48", 48'" followed by a drill removal process 50, 50', 50", 50"', where the drill member 16 is removed from the blind hole 10' in the bone 4. The drill removal process 50, 50', 50", 50'" is followed by a fluid supplementation process 52, 52', 52", 52"', where a cooling fluid is supplied to the hole 10' in the bone 4 in order to cool the bone 4.

Fig. 13 shows four sequence of equal length. The first comprises a drilling process 48 followed by a drill removal process 50 that is followed by a fluid supplementation process 52. The first sequence is followed by a second sequence comprises a drilling process 48' followed by a drill removal process 50' followed by a fluid supplementation process 52'. The second sequence is followed by a third sequence comprises a drilling process 48" followed by a drill removal process 50" that is followed by a fluid supplementation process 52". The third sequence is followed by a fourth sequence comprises a drilling process 48'" followed by a drill removal process 50"' followed by a fluid supplementation process 52"'.

The method according to the disclosure may comprise less than four sequences or more than four sequences. Moreover, the duration of the sequences may vary.

During the drilling process 48, 48', 48", 48"' bone tissue is removed by the drill member. By breaking the drilling process up into several sequences the temperature can be kept down. The drill removal process 50, 50', 50", 50'" facilitates removal pieces of tissue from the drilling process. Finally, the fluid supplementation process 52, 52', 52", 52'" makes it possible to provide a new fluid supplementation between the drilling process 48, 48', 48", 48"'.

Fig. 14 illustrates a graded probe 140 according to an embodiment. The graded probe 140 is adapted to measure depth of the incision hole 10 in the soft tissue 2. The graded probe includes a plurality of markings 142 representing distance in mm from a distal end 144 of the probe, the distal end being adapted to rest against the bone surface 56 that interfaces with the soft tissue. The markings represents distances such as between 8 mm - 16 mm such as 9 mm, 9.5 mm, 12 mm, 13 mm, 14 mm, 16mm etc.. Other finer markings are also possible and within the scope of this disclosure. When the probe is inserted into the hole and the distal end of the probe rests against bone-skin interface, then the marking 146 aligning with the skin surface 42 gives a reading of the depth of the hole in the soft tissue. The reading thus gives an indication of the thickness of the soft tissue at the punch location, allowing a more accurate choice of abutment of appropriate length. Such probes may be made of stainless steel, plastic or any other suitable material.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

Accordingly, the scope should be judged in terms of the claims that follow.

## Claims

1. A drill guide (8, 36, 38) for installing an implant (26) in a blind hole (10') in a bone (4) under a soft tissue (2), the drill guide (8, 36, 38) comprising
a cylindrical portion (44) comprising a cylindrical canal (14) extending along a longitudinal axis (X) of the cylindrical portion (44), the cylindrical canal (14) being adapted to receive a drill member (16) for drilling the blind hole (10') in the bone (4); and
a flange (12), at a proximal end section (66) of the drill guide (8, 36, 38), extending perpendicular or substantially perpendicular to the longitudinal axis (X) of the cylindrical portion (44); wherein
the drill guide (8, 36, 38) has a length (L₁) that is equal or greater than thickness (T) of the soft tissue (2) and a distal end (72) of the drill guide (8, 36, 38), when inserted in a hole (10), is adapted to rest against a bone surface (56) that interfaces with the soft tissue.

2. The drill guide (8) according to claim 1, wherein an outer cylindrical wall (62) of the drill guide (8) abuts the soft tissue (2) when inserted and an inner cylindrical wall (60) guides the drill member (16) for generating the blind hole (10').

3. The drill guide (8) according to claim 1, wherein the flange (12) further includes at least one grip (122) at a periphery of the flange, the grip being adapted to handle the drill guide and to push the drill guide in an incision hole or for making the incision hole if the drill guide includes blade(s).

4. The drill guide (8) according to any of the preceding claims, further comprising
at least one cooling channel (58) comprising at least one proximal channel port (64) positioned at the proximal end section (66) and at least one distal channel port (68) positioned at a distal end section (70) of the drill guide (8); wherein
the at least one proximal channel port (64) is adapted to receive a cooling fluid, the at least one cooling channel (58) is adapted to transmit the cooling fluid from the at least one proximal channel port (64) to the at least one distal channel port (68), which is adapted to expel the cooling fluid out of the at least one cooling channel (58).

5. The drill guide (8) according to any of the preceding claims, wherein the at least one cooling channel is
positioned between the inner cylindrical wall (60) and the outer cylindrical wall (62) of the drill guide (8); and/ or
positioned peripheral to the outer cylindrical wall (62) of the drill guide, the outer peripheral surface (126) of the cooling channel abutting the soft tissue; and/ or
positioned peripheral to the inner cylindrical wall (60) of the drill guide, the inner peripheral surface (124) of the cooling channel guiding the drill member (16).

6. The drill guide (8) according to any of the preceding claims, wherein the distal end section (70) of the drill guide (8) comprises a sharp blade (128) at the distal end (72) with or without spatially separated longitudinal (74) and/ or circular (76) blades along length of the distal end section (70), the blade and/ or blades being selected from a group consisting of regular edge, serrated edge and a combination thereof

7. The drill guide (8) according to any of the preceding claims, wherein the drill guide comprises
at least one opening (120) at the proximal end section (66) of the drill guide, the at least one opening being adapted to expel bone debris during drilling; and/ or
a plurality of spatially separated ribs (80) on an upper surface (82) of the flange (12), each rib of the plurality of ribs comprising a first end (84) in immediate proximity to the drill entry port (86) and a second end (88) at a predetermined distance from the drill entry port (86) of the drill guide (8).

8. The drill guide (8) according to any of the preceding claims, wherein a drill stop member (92) of the drill member (16) is adapted to rest against the upper surface (82) of the flange (12) of the drill guide (8) or top (132) of the plurality of spatially separated ribs (80) during drilling in order to generate desired depth of the blind hole (10') in the bone (4).

9. The drill guide (8) according to any of the preceding claims, wherein the at least one grip (122) is perpendicular or substantially perpendicular to the upper surface (82) of the flange (12).

10. The drill guide (8) according to any of the preceding claims, wherein the at least one grip (122) includes a grip enhancers (134, 136) at a grip surface (138)

11. A healing cap (112) comprising
a flexible inner periphery (100) defining an aperture (102) adapted to allow an attachment end (104) of a top portion (32) of an abutment (30) to pass through the aperture (102), the attachment end (104) being adapted to attach to a processing unit; and
an outer periphery (106), which in combination with the flexible inner periphery (100) defines an upper surface (108) and a lower surface (110) of the healing cap (112), the lower surface (110) facing the skin (42) and the upper surface facing away from the skin (42).

12. The healing cap (112) according to claim 9, further comprising a plurality of evenly or unevenly spaced ridges (114) at the lower surface (110) of the healing cap, a distal end (116) of at least the plurality of ridges (114) proximal to the aperture (102) being adapted to abut the skin and to create pockets (118) of lower surface sections that are at a distance from the skin.

13. The healing cap (112) according to any of the preceding claims, wherein an inner periphery section (122) is thicker than an outer periphery section (124), the variation in thickness being selected from a group consisting of a gradual thickness change, a sudden thickness change, a stepped thickness change, and a combination thereof.

14. A kit comprising at least one of:
a punch adapted to punch a cylindrical hole (10) and providing an incision hole (10) in soft tissue (2) by pressuring a sharp blade of a cylindrical hollow punch member (6) through the soft tissue (2);
a drill guide (8, 36, 38), according to any of the claims 1 to 10, comprising a cylindrical canal (14) being adapted to receive a drill member (16) for drilling a blind hole (10') in the bone (4);
a drill guide (8, 36, 38), according to any of the claims 1 to 10, comprising a cylindrical canal (14) being adapted to receive a drill member (16) for drilling a blind hole (10') in the bone (4); the drill guide further comprising a sharp blade at a distal end with or without spatially separated longitudinal and/ or circular blades along length of the distal end section, the blades being adapted to provide an incision hole (10) in soft tissue (2);
a drill member, according to any of the claims 8-10, adapted to generate the blind hole (10') while being guided through the cylindrical canal (14) of the drill guide (8, 36, 38);
a healing cap, according to any of the claims 11-13, adapted to allow attaching a processing unit to an abutment with the healing cap abutting the skin;
an abutment (30) adapted to be installed in the hole (10) abutting the soft tissue (2); and
an implant (26) adapted to be installed in a blind hole (10') in a bone (4) under a soft tissue (2).

15. A method for installing an implant, the method comprising
punching a cylindrical hole (10) and providing an incision hole (10) in soft tissue (2) by pressuring a sharp blade of a cylindrical hollow punch member (6) or a sharp blade of the drill guide (8) through a soft tissue (2);
pulling out the cylindrical hollow punch member (6) or retaining the drill guide in postion;
removing the punched out soft tissue from the hole (10);
inserting the drill guide (8) into the hole (10) if the cylindrical hollow punch member (6) is used to make the incision hole (10);
inserting the drill member (6) in the hole (10), the insertion being guided by the inserted drill member;
drilling a blind hole (10'), using the inserted drill member (6), in the underlying bone tissue (4); and
anchoring the implant (26) in the blind hole (10').
